# EUROPEAN PATENT APPLICATION

(11) **EP 3 988 009 A1**
(43) Date of publication of application: **27.04.2022**
(21) Application number: 20202901.3
(22) Date of filing: 20.10.2020
(51) Int. Cl.: A61B 5/00, A61B 5/11, A61B 5/16, G06N 3/08, G16H 40/67

(54) **METHOD AND SYSTEM FOR AUTOMATICALLY MONITORING AND DETERMINING THE QUALITY OF LIFE OF A PATIENT**

(71) Applicant: Fresenius Medical Care Deutschland GmbH, 61352 Bad Homburg (DE)
(72) Inventor: SCHERER, Lena, 76131 Karlsruhe (DE); BOTLER, Max, 13189 Berlin (DE)
(74) Representative: Ricker, Mathias

(57) **Abstract**

A computer-implemented method for automatically monitoring and determining the quality of life of a patient, comprises the steps of a) acquiring therapy data concerning a medical therapy that is performed by a medical device on the patient, and b) determining at least one quantifiable score which can be associated with the quality of life of the patient based on the therapy data.

## Description

The present invention relates to computer-implemented method and a system which automatically monitor and determine the quality of life of a patient.

Nowadays, patients who are dependent on life-saving medical therapies due to chronic diseases benefit from the possibility of being able to carry out the life-saving medical therapies completely or at least partially at home. Especially, in the case of chronic kidney diseases, patients often have the option of receiving necessary dialysis therapies, such as peritoneal dialysis, hemofiltration, hemodialysis and/or hemodiafiltration, also outside of clinics. This kind of therapy usually offers patients the great advantage that they no longer need to make long journeys to dialysis clinics, enabling them to make their daily lives as self-sufficient as possible. This kind of therapy is therefore extremely beneficial for many patients, but can also be associated with health risks, as patients are not usually monitored continuously during medical therapies.

Patients who receive their medical therapy in clinics are subject to closer observation during medical therapy, but, just like patients who receive their medical therapy at home, they are usually on their own in their own homes. This is especially true for those patients who live alone in their homes. Since medical therapies such as dialysis therapy are an additional physical burden for patients, the quality of life of patients can be significantly impaired.

From a medical point of view, various scores can be used nowadays in order to quantify the quality of life of patients, wherein the scores are mostly determined by means of clinical questionnaires the patients have to fill in. Examples of scores to quantify the quality of life of patients include the Karnofsky Performance Score, Zubrod Score, Lansky Score, Kidney Disease Quality of Life (KDQOL), Quality-Adjusted Life Years, Patient-Reported Outcome Measures (PROMs), Patient-Reported Experience Measures (PREMs), CHOICE Health Experience Questionnaire (CHEQ), Dialysis Quality of Life (DIA-QOL), Dialysis Symptom Index, RAND-36 and/or SF-36.

Since a low quality of life usually has a negative effect on the success of the medical therapy and, in the worst case, can even lead to a fundamental rejection of a vital medical therapy by the patient, the continuous determination and monitoring of at least one score that allows conclusions to be drawn about the quality of life of dialysis patients is of great importance. Patients with correspondingly low scores and an associated low quality of life also perceive medical therapies as a particularly heavy physiological and psychological burden, so that negative feedback can lead to a further deterioration in the patient's condition. In addition, it can be observed that patients with low scores are more often affected by complications and a higher mortality rate. From a medical point of view, close monitoring of patients, even beyond the actual medical therapy times, is therefore more than desirable.

However, the determination of a score associated with quality of life using clinical questionnaires has the disadvantage for patients that additional time, mostly in clinics, must be planned for. Specifically, for home therapy patients this means a regular additional load, which should not actually be necessary due to the home therapy. Patients who receive their medical therapy in a clinic, on the other hand, must also plan additional time for the processing of the questionnaires. Due to the frequent travel activity, this can also lead to a further load on the part of the patients.

It is therefore an object of the present invention to solve the present technical problem and to provide a computer-implemented method as well as a system which make it possible to monitor and determine the quality of life of patients in detail and automatically.

The object is achieved by the subject matter of the independent claims. Further preferred embodiments of the invention are defined in the dependent claims.

A computer-implemented method for automatically monitoring and determining the quality of life of a patient according to one embodiment comprises the steps of a) acquiring therapy data concerning a medical therapy that is performed by a medical device on the patient, and b) determining at least one quantifiable score which can be associated with the quality of life of the patient based on the therapy data.

Since the score which can be associated with the quality of life of the patient is automatically determined, the load on the part of the patient can be considerably reduced.

Preferably, the therapy data concerning the medical therapy that is performed by the medical device is acquired by one of at least one medical device, including the medical device which performs the medical therapy on the patient, and/or at least one further device.

In this case, the at least one medical device and the at least one further device may each comprise at least one data communication unit, wherein the at least one medical device and the at least one further device can exchange data through the respective data communication units.

The data exchange between the at least one medical device and the at least one further device may be performed directly, for example by establishing an ad hoc connection of the data communication units of the respective devices, and/or by means of a central network gateway unit, which can also be part of one of the data communication units of the at least one medical device and/or of the at least one further device and which allows, for example, additional access to a wide area network (WAN) such as the Internet. In a preferred embodiment, at least one of the at least one further device can be identical to the network gateway unit and can, for example, function as an access point within the medical system.

The at least one data communication unit of the at least one medical device and the data communication unit of the at least one further device may be configured to perform wireless and/or wired data communication. Accordingly, the data exchange between the at least one medical device and the at least one further device can be performed using any combination of wireless and/or wired data communication. Wireless data communication can be established by modulating electromagnetic carrier signals, e.g. microwaves, radio waves, infrared, visible light, and/or by modulating acoustic carrier signals, e.g. infra-sound, audible sound and/or ultrasound. For performing wireless transmission in the frequency range of radio waves technologies such as Bluetooth, WLAN, 3G, LTE, 4G, 5G, ZigBee, NFC, Wibree and/or WiMAX may be used. Technologies in the optical spectrum are for example IrDA, Free-Space Optical communication (FSO), LiFi and/or the transmission by means of one- or two-dimensional bar codes and/or dynamic flicker codes, which can be displayed by means of a display of a first medical device and/or a first further device and can be detected by means of optical detection means of a second medical device and/or a second further device for the purpose of data exchange. Wired transmission paths may include electrical wires, e.g. Ethernet, D-LAN, PLC, and/or optical fibers

The data communication between the medical devices and/or the further devices can be performed within a local network and/or partly or completely via a wide area network (WAN), such that a data exchange between further external medical devices and/or further external further devices is possible. In this case, the data communication can be processed by a central server which is in communication with clinics, physicians, therapy centers and/or housing units. In another embodiment, the data communication can also be performed by means of a decentralized network infrastructure, for example by means of a peer to peer network. Accordingly, the medical devices and/or the further devices may be located at different locations. For example, one or more medical devices, in particular medical therapeutic devices, can be positioned in a clinic and/or in a therapy center, whereas the further devices are positioned in a housing unit of the patient. This embodiment is especially suited for patients who receive their medical therapy outside their housing units. The term "housing units" comprises all kinds of dwellings, which are inhabited by physicians, patients, nurses and/or technicians. In the case of patients, the housing units can be for example privately used apartments, houses and/or care facilities.

According to one embodiment, step a) comprises acquiring therapy data concerning a fluid therapy that is performed by the medical device on the patient.

In this case, the fluid therapy may comprise a peritoneal dialysis, a hemofiltration, a hemodialysis, a hemodiafiltration, an apheresis, a plasmapheresis and/or an oxygenator therapy.

In general, the term "therapy data" comprises all data that can usually be collected by the medical device or another device before, during or after a medical therapy.

Preferably, step a) comprises acquiring data concerning a duration and a kind of the medical therapy, a number and a kind of occurred alarms during the medical therapy, indications due to technical malfunctions and/or medical complications output by the medical device, and/or therapy-specific indices.

In this case, step a) may comprise acquiring therapy data concerning a kidney replacement therapy that is performed by the medical device on the patient, and the therapy-specific indices may include a clearance, a Kt/V, an ultrafiltration volume, and/or a proportion of the urea level before, during and/or after the medical therapy. Alternatively or additionally, step a) may also comprise patient data from possible psychotherapies or other treatments the patient is simultaneously receiving.

According to an embodiment, the method further comprises acquiring physiological data of the patient, and/or of other people and/or of other species in the environment of the patient, wherein step b) comprises determining the at least one quantifiable score which can be associated with the quality of life of the patient based on the therapy data and the physiological data.

According to this embodiment, language, behavior, facial expressions and/or gestures of patients, family members, or the behavior of pets or other species, can be used to draw conclusions about the patient's quality of life. Furthermore, data concerning the environment of the patient can be acquired, e.g. the condition of the rooms and/or the location and condition of the patient's furnishings, so that signs of neglect can be detected in time. For example, the state of order of the apartment can be captured by means of an image using artificial intelligence to obtain additional information about the patient's behavior, which allows conclusions to be drawn about his quality of life.

In this embodiment, acquiring physiological data of the patient may comprise acquiring a weight, an oxygen saturation, a heart rate, a blood pressure, a body temperature, a breathing rate, an eye or eyelid movements, a conductivity of the skin and/or a liquid state of the patient, and acquiring physiological data of the patient and/or of other people and/or of other species in the environment of the patient may comprise acquiring a size, shape and/or movement of the pupils, blinking, fixations and/or saccades, facial expression, skin pallor, speech, speech volume, speech speed, prosody, tones and/or sounds of the patient and/or of other people and/or of other species in the environment of the patient.

According to an embodiment, the method further comprises acquiring sound, image and/or video data of the patient or his environment, wherein step b) comprises determining the at least one quantifiable score which can be associated with the quality of life of the patient based on the physiological data and/or at least one of the sound, the image and the video data of the patient or his environment, and the therapy data.

In this case, acquiring the therapy data and/or acquiring the sound, image and/or video data of the patient or his environment can be performed temporally punctual, intermittent or continuous.

Preferably, the therapy data and/or the physiological data and/or the at least one of the sound, the image and the video data of the patient or his environment is acquired by one of at least one medical device, including the medical device which performs the medical therapy on the patient, and/or at least one further device, wherein the at least one medical device and/or the at least one further device comprise at least one detection means configured to acquire the therapy data and/or the physiological data and/or the sound, image and/or video data of the patient or his environment.

In this case, the at least one detection means may comprise a still camera, a video camera, an infrared camera, a thermal imaging camera, a stereoscopic camera, a microphone, a radar sensor, an ultrasonic sensor, a pressure sensor, an impedance analyzer, an infrared sensor, a gyroscopic sensor, a piezoelectric sensor and/or another sensor by which gravity fields, electric fields, magnetic fields and or electromagnetic fields can be measured.

In a preferred embodiment, the at least one medical device and/or the at least one further device is a smart device, in particular a smart phone, a tablet, a smart speaker, a smart watch, a smart TV, a game console, a notebook, a convertible, a Smart Home, a fitness wristband, an action camera, a VR/AR glass or any other device that enables communication with the Internet of Things.

Preferably, determining the at least one quantifiable score which can be associated with the quality of life of the patient based on at least one of the sound, the image and the video data of the patient or his environment, and the therapy data and/or the physiological data comprises processing the sound and/or image data using Natural Language Processing, Sound Processing, Eye Tracking and Processing, Sentiment Analysis using any combination of Convolutional Neural Networks, Transfer Learning, Recurrent Neural Networks, Long-Short-Term Memory Networks, Reinforcement Learning, Auto Encoder and/or Generative Adversarial Networks.

According to an embodiment, the method further comprises transmitting the determined at least one quantifiable score to a device accessible by an authorized person and outputting a notification about the determined at least one quantifiable score using the device and/or processing the determined at least one quantifiable score by a computer program. The device accessible by an authorized person can be one device of the further devices.

Authorized persons are, for example, patients themselves, attending physicians, nurses and/or other clinicians. Furthermore, authorized relatives, e.g. parents, grandparents, children, grandchildren, siblings, or service providers, technicians and/or authorized companies can also be notified of the patient's condition. It is also conceivable that one and/or more scores are further processed by one or more computer programs. These computer programs can also be part of the medical system, which includes the at least one medical device and the at least one further device, and installed on one or more of the data processing units of the medical devices and/or the further devices. Alternatively, or additionally, these computer programs can be implemented on data processing units external to the medical system, for example on clinic servers, company servers and/or cloud servers of corresponding providers.

In this embodiment, the method preferably further comprises determining whether one or more of the determined at least one quantifiable score exceeds an upper threshold value and/or is lower than a lower threshold value, and transmitting the one or more of the determined at least one quantifiable score to the device accessible by the authorized person and outputting the notification about the one or more of the determined at least one quantifiable score using the device, and/or processing the one or more of the determined at least one quantifiable score by the computer program, if it is determined that the one or more of the determined at least one quantifiable score exceeds the upper threshold value and/or is lower than the lower threshold value.

In a preferred embodiment, the method further comprises determining, based on the acquired physiological data and/or the determination whether one or more of the determined at least one quantifiable score exceeds the upper threshold value and/or is lower than the lower threshold value, whether the patient is in a critical condition, wherein the determined at least one quantifiable score and the one or more of the determined at least one quantifiable score which exceeds the upper threshold value and/or is lower than the lower threshold value, respectively, is transmitted to the device accessible by the authorized person and the notification about the determined at least one quantifiable score and the one or more of the determined at least one quantifiable score which exceeds the upper threshold value and/or is lower than the lower threshold value, respectively, is output using the device and/or the determined at least one quantifiable score and the one or more of the determined at least one quantifiable score which exceeds the upper threshold value and/or is lower than the lower threshold value, respectively, is processed by the computer program, if it is determined that the patient is in the critical condition.

The critical condition of the patient includes e.g. a case where there is an immediate risk to the patient of suffering a heart attack. In this case, the critical condition can be determined based on specific physiological data such as high blood pressure, shallow/no breathing, very low/high pulse.

The determined at least one quantifiable score and the one or more of the determined at least one quantifiable score which exceeds the upper threshold value and/or is lower than the lower threshold value, respectively, may be transmitted to the device accessible by the authorized person and the notification about the determined at least one quantifiable score and the determined one or more of the determined at least one quantifiable score, respectively, may be output using the device and/or the determined at least one quantifiable score and the one or more of the determined at least one quantifiable score which exceeds the upper threshold value and/or is lower than the lower threshold value, respectively, may be processed by the computer program only if a corresponding consent given by the patient is received.

In this case, it can be made clear to the patient based on an acoustical, optical and/or haptic signal that notices/messages/alarms are to be transmitted. For being able to receive the consent given by the patient, the at least one medical device and/or the at least one further device may comprise a communication means which allows them to interact with the patient visually, acoustically or haptically. The communication means can, e.g., include screens, loudspeakers, projectors, stereoscopic screens, (piezoelectric) actuators, Eccentric Rotating Mass actuators and/or ultrasonic emitters. In a preferred embodiment, the communication means is configured such that transmitting of the notice/message/alarm can be approved or denied by the patient, by means of a voice command, a gesture, by pressing a button or a touch-sensitive display and/or a virtual button using haptic feedback.

The transmitting of the notices/messages/alarms in the case of specific scores can be performed using a respective data communication unit of one or more medical devices and/or further connected devices. Accordingly, it is also conceivable that at first and external data processing unit receives one or more scores and initiates the transmission of the notices/messages/alarms based on predetermined threshold values. Examples of servers that are operated by clinics and/or companies. A further possibility are various cloud services (AWS, MS Azure, Google Cloud), which can be implemented in an alternative form in any combination with the implementations described above.

In a preferred embodiment, determining the at least one quantifiable score is performed using machine learning and/or deep learning and/or classical data processing algorithms of statistics.

In this case, step b) may be performed using an artificial neural network having an initial set of parameters, wherein the method further comprises comparing a quantifiable score, which can be associated with the quality of life of the patient and which is determined based on the first set of parameters, with a predetermined test score, minimizing a mathematical distance between the quantifiable score, which can be associated with the quality of life of the patient and which is determined based on the first set of parameters, and the predetermined test score, and deriving a rule, which specifies an adjustment of the first set of parameters, based on the minimized mathematical distance.

A medical system according to one embodiment for automatically monitoring and determining the quality of life of a patient comprises at least one medical device having at least one data communication unit and configured to perform a medical therapy on the patient, at least one further device having at least one data communication unit, and at least one computer program, wherein the at least one medical device and the at least one further device are configured to exchange data directly or indirectly with each other by means of the respective data communication unit, the at least one medical device and/or the at least one further device are configured to acquire therapy data concerning the medical therapy that is performed by the at least one medical device on the patient, and the at least one computer program comprises instructions which, when the program is executed by a computer, causes the computer to determine at least one quantifiable score which can be associated with the quality of life of the patient based on the therapy data.

The data exchange between the at least one medical device and the at least one further device may be performed directly, for example by establishing an ad hoc connection of the data communication units of the respective devices, and/or by means of a central network gateway unit, which can also be part of one of the data communication units of the at least one medical device and/or of the at least one further device and which allows, for example, additional access to a wide area network (WAN) such as the Internet. In a preferred embodiment, at least one of the at least one further device can be identical to the network gateway unit and can, for example, function as an access point within the medical system.

The at least one data communication unit of the at least one medical device and the data communication unit of the at least one further device may be configured to perform wireless and/or wired data communication. Accordingly, the data exchange between the at least one medical device and the at least one further device can be performed using any combination of wireless and/or wired data communication. Wireless data communication can be established by modulating electromagnetic carrier signals, e.g. microwaves, radio waves, infrared, visible light, and/or by modulating acoustic carrier signals, e.g. infra-sound, audible sound and/or ultrasound. For performing wireless transmission in the frequency range of radio waves technologies such as Bluetooth, WLAN, 3G, LTE, 4G, 5G, ZigBee, NFC, Wibree and/or WiMAX may be used. Technologies in the optical spectrum are for example IrDA, Free-Space Optical communication (FSO), LiFi and/or the transmission by means of one- or two-dimensional bar codes and/or dynamic flicker codes, which can be displayed by means of a display of a first medical device and/or a first further device and can be detected by means of optical detection means of a second medical device and/or a second further device for the purpose of data exchange. Wired transmission paths may include electrical wires, e.g. Ethernet, D-LAN, PLC, and/or optical fibers.

The data communication between the medical devices and/or the further devices can be performed within a local network and/or partly or completely via a wide area network (WAN), such that a data exchange between further external medical devices and/or further external further devices is possible. In this case, the data communication can be processed by a central server which is in communication with clinics, physicians, therapy centers and/or housing units. In another embodiment, the data communication can also be performed by means of a decentralized network infrastructure, for example by means of a peer to peer network. Accordingly, the medical devices and/or the further devices may be located at different locations. For example, one or more medical devices, in particular medical therapeutic devices, can be positioned in a clinic and/or in a therapy center, whereas the further devices are positioned in a housing unit of the patient. This embodiment is especially suited for patients who receive their medical therapy outside their housing units. The term "housing units" comprises all kinds of dwellings, which are inhabited by physicians, patients, nurses and/or technicians. In the case of patients, the housing units can be for example privately used apartments, houses and/or care facilities.

According to an embodiment, the at least one medical device is configured to perform a fluid therapy on the patient.

In this case, the at least one medical device may be configured to perform a peritoneal dialysis, a hemofiltration, a hemodialysis, a hemodiafiltration, an apheresis, a plasmapheresis and/or an oxygenator therapy on the patient.

In general, the term "therapy data" comprises all data that can usually be collected by the medical device or another device before, during or after a medical therapy.

According to an embodiment, the at least one medical device and/or the further device are configured to acquire therapy data concerning a duration and a kind of the medical therapy, a number and a kind of occurred alarms during the medical therapy, indications due to technical malfunctions and/or medical complications output by the medical device, and/or therapy-specific indices.

In this regard, specifically the acquiring of the therapy-specific indices is useful in order to be able to quantify a success, an effectiveness and/or a trend of the medical therapy.

In this embodiment, the medical device may be configured to perform a kidney replacement therapy on the patient, and the therapy-specific indices may include a clearance, a Kt/V, an ultrafiltration volume, and/or a proportion of the urea level before, during and/or after the medical therapy.

In other embodiments, the at least one medical device may include infusion pumps, pacemakers and/or respirators. Furthermore, the at least one medical device can include medical measurement devices such as blood pressure monitors, pulse monitors, scales, impedance analyzers, electrocardiographs, electroencephalographs, X-ray machines, magnetic resonance tomographs, computer tomographs and/or positron emission tomographs.

In a preferred embodiment, the at least one medical device and/or the at least one further device are configured to acquire physiological data of the patient, and/or of other people and/or of other species in the environment of the patient, wherein the at least one computer program comprises instructions which, when the program is executed by the computer, causes the computer to determine the at least one quantifiable score which can be associated with the quality of life of the patient based on the therapy data and the physiological data.

According to this embodiment, language, behavior, facial expressions and/or gestures of patients, family members, or the behavior of pets or other species, can be used to draw conclusions about the patient's quality of life. Furthermore, data concerning the environment of the patient can be acquired, e.g. the condition of the rooms and/or the location and condition of the patient's furnishings, so that signs of neglect can be detected in time. For example, the state of order of the apartment can be captured by means of an image using artificial intelligence to obtain additional information about the patient's behavior, which allows conclusions to be drawn about his quality of life.

In this preferred embodiment, the at least one medical device and/or the at least one further device may be configured to acquire a weight, an oxygen saturation, a heart rate, a blood pressure, a body temperature, a breathing rate, an eye or eyelid movements, a conductivity of the skin and/or a liquid state of the patient, and to acquire a size, shape and/or movement of the pupils, blinking, fixations and/or saccades, facial expression, skin pallor, speech, speech volume, speech speed, prosody, tones and/or sounds of the patient and/or of other people and/or of other species in the environment of the patient.

Furthermore, in this preferred embodiment, the at least one medical device and/or the at least one further device may be configured to acquire sound, image and/or video data of the patient or his environment, and the at least one computer program may comprise instructions which, when the program is executed by the computer, causes the computer to determine the at least one quantifiable score which can be associated with the quality of life of the patient based on the physiological data and/or and at least one of the sound, the image and the video data of the patient or his environment, and the therapy data.

In this case, the medical device and/or the further device may be configured to acquire the therapy data and/or the sound, the image and the video data of the patient or his environment temporally punctual, intermittent or continuous.

Furthermore, the at least one computer program may comprise instructions which, when the program is executed by the computer, causes the computer to determine the at least one quantifiable score which can be associated with the quality of life of the patient based on at least one of the sound, the image and/or the video data of the patient or his environment, and the therapy data and/or the physiological data using processing the sound and/or image data using Natural Language Processing, Sound Processing, Eye Tracking and Processing, Sentiment Analysis using any combination of Convolutional Neural Networks, Transfer Learning, Recurrent Neural Networks, Long-Short-Term Memory Networks, Reinforcement Learning, Auto Encoder and/or Generative Adversarial Networks.

Preferably, the at least one further device comprises a device accessible by an authorized person, the at least one computer program comprises instructions which, when the program is executed by the computer, causes the computer to transmit the determined at least one quantifiable score to the device accessible by the authorized person and/or to another computer program of the at least one computer program in order to process the determined at least one quantifiable score by the other computer program, and the device accessible by the authorized person is configured to output a notification about the received determined at least one quantifiable score.

Authorized persons are, for example, patients themselves, attending physicians, nurses and/or other clinicians. Furthermore, authorized relatives, e.g. parents, grandparents, children, grandchildren, siblings, or service providers, technicians and/or authorized companies can also be notified of the patient's condition. It is also conceivable that one and/or more scores are further processed by one or more computer programs. These computer programs can also be part of the medical system, which includes the at least one medical device and the at least one further device, and installed on one or more of the data processing units of the medical devices and/or the further devices. Alternatively, or additionally, these computer programs can be implemented on data processing units external to the medical system, for example on clinic servers, company servers and/or cloud servers of corresponding providers.

Furthermore, the at least one computer program may comprise instructions which, when the program is executed by the computer, causes the computer to determine whether one or more of the determined at least one quantifiable score exceeds an upper threshold value and/or is lower than a lower threshold value, to transmit the one or more of the determined at least one quantifiable score to the device accessible by the authorized person and/or to the other computer program in order to process the one or more of the determined at least one quantifiable score by the other computer program, if it is determined that the one or more of the determined at least one quantifiable score exceeds the upper threshold value and/or is lower than the lower threshold value.

Furthermore, the at least one computer program may comprise instructions which, when the program is executed by the computer, causes the computer to determine, based on the acquired physiological data and/or the determination whether one or more of the determined at least one quantifiable score exceeds the upper threshold value and/or is lower than the lower threshold value, whether the patient is in a critical condition, and to transmit the determined at least one quantifiable score and the one or more of the determined at least one quantifiable score which exceeds the upper threshold value and/or is lower than the lower threshold value, respectively, to the device accessible by the authorized person and/or to the other computer program in order to process the determined at least one quantifiable score and the one or more of the determined at least one quantifiable score which exceeds the upper threshold value and/or is lower than the lower threshold value, respectively by the other computer program, if it is determined that the patient is in the critical condition.

The critical condition of the patient includes e.g. a case where there is an immediate risk to the patient of suffering a heart attack. In this case, the critical condition can be determined based on specific physiological data such as high blood pressure, shallow/no breathing, very low/high pulse.

In addition, the at least one computer program may comprise instructions which, when the program is executed by the computer, causes the computer to transmit the determined at least one quantifiable score and the one or more of the determined at least one quantifiable score which exceeds the upper threshold value and/or is lower than the lower threshold value, respectively, to the device accessible by the authorized person and/or to the other computer program in order to process the determined at least one quantifiable score and the one or more of the determined at least one quantifiable score which exceeds the upper threshold value and/or is lower than the lower threshold value, respectively by the other computer program only if a corresponding consent given by the patient is received by one of the further devices.

In this case, it can be made clear to the patient based on an acoustical, optical and/or haptic signal that notices/messages/alarms are to be transmitted. For being able to receive the consent given by the patient, the at least one medical device and/or the at least one further device may comprise a communication means which allows them to interact with the patient visually, acoustically or haptically. The communication means can, e.g., include screens, loudspeakers, projectors, stereoscopic screens, (piezoelectric) actuators, Eccentric Rotating Mass actuators and/or ultrasonic emitters. In a preferred embodiment, the communication means is configured such that transmitting of the notice/message/alarm can be approved or denied by the patient, by means of a voice command, a gesture, by pressing a button or a touch-sensitive display and/or a virtual button using haptic feedback.

The transmitting of the notices/messages/alarms in the case of specific scores can be performed using a respective data communication unit of one or more medical devices and/or further connected devices. Accordingly, it is also conceivable that at first an external data processing unit receives one or more scores and initiates the transmission of the notices/messages/alarms based on predetermined threshold values. Examples of servers that are operated by clinics and/or companies. A further possibility are various cloud services (AWS, MS Azure, Google Cloud), which can be implemented in an alternative form in any combination with the implementations described above.

According to a preferred embodiment, the at least one computer program comprises instructions which, when the program is executed by the computer, causes the computer to determine the at least one quantifiable score using machine learning and/or deep learning and/or classical data processing algorithms of statistics.

In this case, the at least one computer program may comprise instructions which, when the program is executed by computer, causes the computer to determine the at least one quantifiable score using an artificial neural network having an initial set of parameters, compare a quantifiable score, which can be associated with the quality of life of the patient and which is determined based on the first set of parameters, with a predetermined test score, minimize a mathematical distance between the quantifiable score, which can be associated with the quality of life of the patient and which is determined based on the first set of parameters, and the predetermined test score, and derive a rule, which specifies an adjustment of the first set of parameters, based on the minimized mathematical distance.

The predetermined test score can be, for example, generated based on a combination of therapeutic data, patient monitoring and clinical questionnaires, and the mathematical distance can be, for example, a p-norm. Methods known from the prior art such as different gradient methods can be used for this minimizing of the mathematical distance.

Preferably, the at least one medical device and/or the at least one further device comprise a communication means which allows them to interact with the patient visually, acoustically or haptically. The communication means can, e.g., include screens, loudspeakers, projectors, stereoscopic screens, (piezoelectric) actuators, Eccentric Rotating Mass actuators and/or ultrasonic emitters.

According to a preferred embodiment, the at least one medical device and/or the at least one further device comprise at least one detection means configured to acquire the therapy data and/or the physiological data and/or the sound, image and/or video data of the patient or his environment.

In this case, the at least one detection means may comprise a still camera, a video camera, an infrared camera, a thermal imaging camera, a stereoscopic camera, a microphone, a radar sensor, an ultrasonic sensor, a pressure sensor, an impedance analyzer, an infrared sensor, a gyroscopic sensor, a piezoelectric sensor and/or another sensor by which gravity fields, electric fields, magnetic fields and or electromagnetic fields can be measured.

Furthermore, by means of the data communication units of the medical devices and of the further devices the data, which are acquired by detection means of the medical devices and the further devices, are transmitted to at least one data processing unit. The data communication between the data communication unit and the data processing unit can be performed using any kind of communication of wireless communication and/or wired communication. In one embodiment, the at least one data processing unit can be integrated in at least one medical device and/or at least one further device. In another embodiment, the data processing units are provided in the medical devices, the further devices and/or separate devices, which can be located locally at the patient and/or at a location remote from the patient.

According to an embodiment, the at least one medical device and/or the at least one further device is a smart device, in particular a smart phone, a tablet, a smart speaker, a smart watch, a smart TV, a game console, a notebook, a convertible, a Smart Home, a fitness wristband, an action camera, a VR/AR glass or any other device that enables communication with the Internet of Things.

The at least one computer program and/or the other computer program can be stored in a data processing unit of the at least one medical device or in a data processing unit of the at least further device or in at least one data processing unit external from the at least one medical device and the at least one further device and having at least one data communication unit configured to exchange data directly or indirectly with the at least one medical device and the at least one further device.

In another embodiment, parts of the at least one computer program and/or of the other computer program may be stored in one or more data processing units of the at least one medical device, the at least further device and in at least one data processing unit external from the at least one medical device and the at least one further device and having at least one data communication unit configured to exchange data directly or indirectly with the at least one medical device and the at least one further device.

The data processing unit has volatile and/or non-volatile data memories and at least one processor unit, e.g. a Central Processing Unit (CPU), Graphics Processing Unit (GPU) and/or Intelligent Processing Unit (IPU). The processing unit may also be identical to the data memory and all calculations may be performed in the memory itself and/or future computer architectures, e.g. quantum computers and/or quantum annealers, may be used. Furthermore, the individual components of the data processing unit may be located in several different places. Additionally, it is possible, that the individual components of the data processing unit are located at a plurality of different locations. In one embodiment, the processor units and/or data memories are located in any combination at different locations, whereby, for example, sensitive patient-related data do not leave the patient's home environment, the clinical environment, secure public databases and/or secure corporate databases and thus remain on a first data memory. In addition, only a calculation result that is uncritical in terms of data protection law and is anonymized is sent to a potentially critical data storage device in order to be processed there by another computer program, for example. This technique has already been disclosed and is known in the state of the art as "federated learning".

A system according to one embodiment for adapting a therapy plan for a patient comprises a medical system described above, wherein the at least one computer program comprises instructions which, when the program is executed by the computer, causes the computer to change or optimize a therapy plan of the medical therapy including shortening therapy sessions of the medical therapy and/or changing a kind of the medical therapy depending on the determined at least one quantifiable score and/or the physiological data and/or and at least one of the sound, the image and the video data of the patient or his environment and/or other patient-related data.

In the system, the at least one medical device and/or the at least one further device may comprise the communication means, wherein the at least one computer program may comprise instructions which, when the program is executed by the computer, causes the computer to communicate with the patient by means of a language assistant and/or to offer the patient to actively participate in the design of the medical therapy and/or to offer support by other persons and/or to offer to repair the medical device used for the medical therapy and/or to offer the patient a psychotherapy and/or to administer psychopharmacological drugs and/or to offer additional support by a nurse or a physician during the medical therapy by means of the communication means.

In this case, the at least one computer program may comprise instructions which, when the program is executed by the computer, causes the computer to communicate with the patient by asking questions about his current situation and by receiving a response via the communication means, and to determine the at least one quantifiable score which can be associated with the quality of life of the patient based on the therapy data and/or the physiological data and/or and at least one of the sound, the image and the video data of the patient or his environment and/or the other patient-related data and/or the response.

According to an embodiment, the at least one medical device and/or the at least one further device comprise the communication means, wherein the at least one computer program comprises instructions which, when the program is executed by the computer, causes the computer to communicate by means of the communication means with an authorized person, wherein the authorized person is enabled to acknowledge, modify or refuse the changed or optimized therapy plan of the medical therapy.

Preferably, the at least one medical device and/or the at least one further device comprise the communication means, wherein the at least one computer program comprises instructions which, when the program is executed by the computer, causes the computer to generate multiple different therapy plans by changing or optimizing the therapy plan, and to allow the patient to select one of the multiple different therapy plans by means of the communication means.

Furthermore, the at least one medical device and/or the at least one further device may comprise the communication means, wherein the at least one computer program comprises instructions which, when the program is executed by the computer, causes the computer to generate multiple different therapy plans by changing or optimizing the therapy plan, to allow an authorized person to select one or more of the multiple different therapy plans by means of the communication means, and to allow the patient to select one of the selected of the multiple different therapy plans by means of the communication means.

Preferably, computer programs, which are configured for initiating one of the measures described above, can also make use of machine learning and/or deep learning methods.

A system for automatically determining the quality of life of a patient according to an embodiment comprises a medical system as described above or a system as described above, at least one computer accessible by authorized personal of a clinic and having a data processing unit and a communication unit, at least one computer accessible by an established physician and having a data processing unit and a communication unit, at least one computer accessible by a medical device manufacturer and having a data processing unit and a communication unit, and a central data processing unit having a data processing unit and a communication unit, wherein the at least one medical device and/or the at least one further device are configured to transmit the acquired therapy data, the acquired psychological, the acquired sound and/or image data to the central data processing unit, the at least one computer accessible by an established physician is configured to transmit anamnesis data, current test data and/or patient history data of the patient to the central data processing, the at least one computer accessible by authorized personal of a clinic is configured to transmit clinic data regarding the patient to the central processing unit, the at least one computer accessible by a medical device manufacturer is configured to transmit data concerning the at least one medical device to the central processing unit, the central data processing unit is configured to determine the at least one quantifiable score which can be associated with the quality of the patient based on the therapy data and/or the psychological data and/or the sound data and/or the image data transmitted from the at least one medical device and/or the at least one further device and/or the clinic data regarding the patient transmitted from the at least one computer accessible by authorized personal of a clinic and/or the anamnesis data, current test data and/or patient history of the patient transmitted from the at least one computer accessible by an established physician and/or the data concerning the at least one medical device transmitted from the at least one computer accessible by a medical device manufacturer.

Preferably, the at least one medical device and/or the at least one further device, the at least one computer accessible by an established physician, and the at least one computer accessible by authorized personal of a clinic have access to the at least one quantifiable score which can be associated with the quality of the patient, and which has been determined by the central data processing unit.

Further embodiments and other objects, advantages and features of the present invention will become apparent from the following detailed description of the invention, the illustration of particular embodiments and the accompanying figures, in which
Fig. 1 shows a part of a system for automatically determining the quality of life of a patient according to one embodiment,
Fig. 2 shows a part of a system for automatically determining the quality of life of a patient according to another embodiment, and
Fig. 3 schematically illustrates a system for automatically determining the quality of life of a patient according to an embodiment.

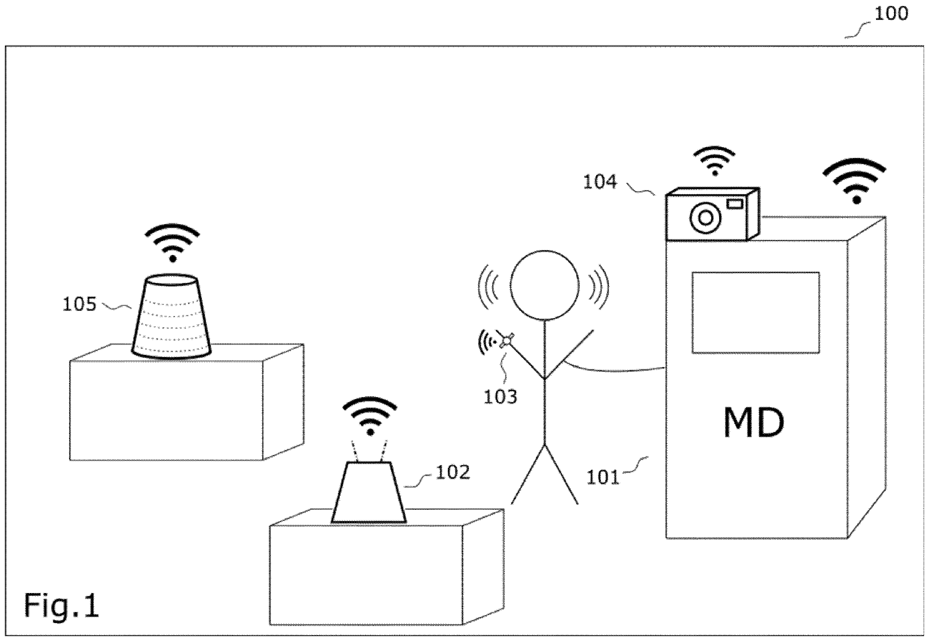

Fig. 1 shows a part of a system for automatically determining the quality of life of a patient according to one embodiment. The system comprises a medical device 101, which is configured to perform a medical therapy, e.g. a fluid therapy, on a patient and which comprises at least one data communication unit (not shown). Furthermore, the system comprises further devices 103, 104, 105, which each comprise at least one data communication unit (not shown), and a network gateway unit 102. By means of the respective data communication units, the medical device 101, the further devices 103, 104, 105, and the network gateway unit 102 can communicate with each other and exchange data. In the present embodiment, the data communication units are configured to perform wireless communication. In other embodiments, at least one of the data communication units may be configured to perform wired communication.

The medical device 101, the further devices 103, 104, 105, and the network gateway unit 102 are located in a home 100, such as an apartment, of the patient. The first further device 104 is a camera configured to acquire image, video and sound data, the second further device 105 is a Smart Speaker configured to acquire sound data and to communicate with the patient by means of a language assistant, and the third further device 103 is a Smartwatch configured to acquire an oxygen saturation and a heart rate of the patient.

During, before and/or after the patient undergoes a medical therapy by means of the medical device 101, sound and image data of the patient are acquired using the camera 104 and the Smart Speaker 105. At the same time, physiological data, namely the oxygen saturation and the heart rate of the patient, are acquired by means of the Smartwatch 103. The sound data, the image data, the physiological data and therapy data obtained from the medical device 101 are then transmitted via the network gateway unit 102 to an external data processing unit (not shown) on which a computer program is installed which determines at least one quantifiable score which can be associated with the quality of life of the patient based on the sound data, the image data, the physiological data and the therapy data.

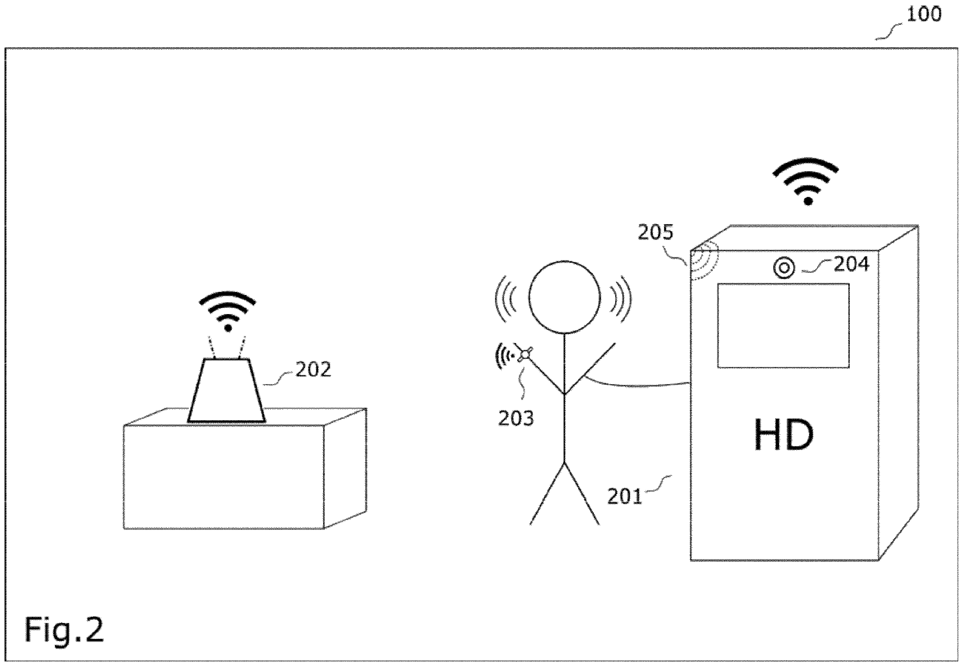

Fig. 2 shows a part of a system for automatically determining the quality of life of a patient according to another embodiment. The system comprises a medical device 201 configured as hemodialysis device comprising a camera 204 and a combination of a microphone and a loudspeaker 205, a further device 203 configured as a Smartwatch, and a network gateway unit 202. The medical device 201 is configured to monitor the patient, i.e. to acquire sound data image date and/or video data of the patient, before, during and/or after the medical system 201 performs a kidney replacement therapy. At the same time, additional physiological data are acquired by means of the Smartwatch 203. The acquired data are then transmitted to an external data processing unit (not shown) via the network gateway unit 202 by means of a wireless transmission. Upon receipt of the acquired data, a computer program installed on the external data processing unit determines at least one quantifiable score which can be associated with the quality of life of the patient based on the data transmitted via the network gateway unit 202.

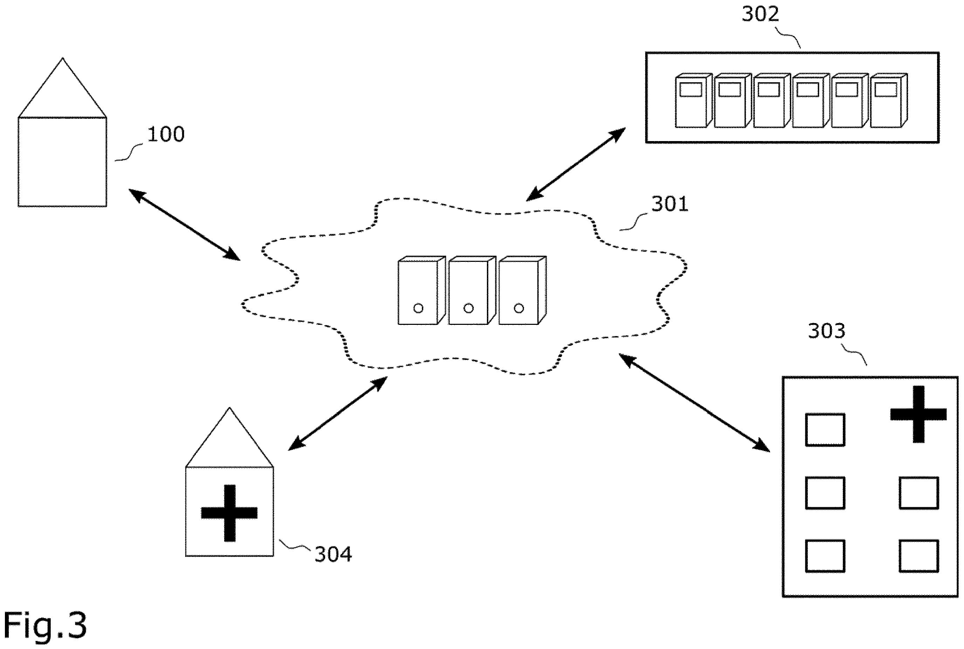

Fig. 3 schematically illustrates a system for automatically determining the quality of life of a patient according to an embodiment. The system comprises at least one medical device (not shown) and at least one further device (not shown), which are located in the patient's home 100, and a central data processing unit 301. The at least one medical device and the at least one further device can exchange data with the central data processing unit 301 by means of a wireless communication. In particular, the at least one medical device and/or the at least one further device can transmit therapy data, physiological data, sound data and/or image data to the central data processing unit 301, and receive from the central data processing unit 301 at least one quantifiable score which can be associated with the quality of life of the patient and which is calculated by a computer program installed on the central processing unit.

The central processing unit 301, in particular the computer program installed thereon, can additionally communicate with established physicians 304, clinics 303 and/or medical device manufacturers 302. Hence, for determining the at least one quantifiable score, the computer program can use additional data such as anamnesis data, current test data and/or patient history of the patient transmitted from the established physicians 304, clinic data transmitted from the clinics 303 and/or data transmitted from the medical device manufacturers 302. Therapy data from clinical therapies and/or tests can also be correlated with the latest information on the medical devices used and thus be included in the calculation of a score. In return, authorized persons, e.g., physicians 304 and/or hospitals 303, and/or computer programs are given access to the at least one score in order to take the corresponding quality of life into account when considering further therapy planning.

## Claims

1. A computer-implemented method for automatically monitoring and determining the quality of life of a patient, comprising the steps of:
a) acquiring therapy data concerning a medical therapy that is performed by a medical device (101, 201) on the patient, and
b) determining at least one quantifiable score which can be associated with the quality of life of the patient based on the therapy data.

2. The method according to claim 1, wherein step a) comprises acquiring therapy data concerning a fluid therapy that is performed by the medical device (101, 201) on the patient, wherein the fluid therapy comprises a peritoneal dialysis, a hemofiltration, a hemodialysis, a hemodiafiltration, an apheresis, a plasmapheresis and/or an oxygenator therapy.

3. The method according to one of the preceding claims, wherein step a) comprises acquiring data concerning a duration and a kind of the medical therapy, a number and a kind of occurred alarms during the medical therapy, indications due to technical malfunctions and/or medical complications output by the medical device (101, 201), and/or therapy-specific indices.

4. The method according to claim 3, wherein step a) comprises acquiring therapy data concerning a kidney replacement therapy that is performed by the medical device (101, 201) on the patient, and the therapy-specific indices include a clearance, a Kt/V, an ultrafiltration volume, and/or a proportion of the urea level before, during and/or after the medical therapy.

5. The method according to one of the preceding claims, further comprising acquiring physiological data of the patient, and/or of other people and/or of other species in the environment of the patient, wherein step b) comprises determining the at least one quantifiable score which can be associated with the quality of life of the patient based on the therapy data and the physiological data.

6. The method according to claim 5, wherein acquiring physiological data of the patient comprises acquiring a weight, an oxygen saturation, a heart rate, a blood pressure, a body temperature, a breathing rate, an eye or eyelid movements, a conductivity of the skin and/or a liquid state of the patient, and acquiring physiological data of the patient and/or of other people and/or of other species in the environment of the patient comprises acquiring a size, shape and/or movement of the pupils, blinking, fixations and/or saccades, facial expression, skin pallor, speech, speech volume, speech speed, prosody, tones and/or sounds of the patient and/or of other people and/or of other species in the environment of the patient.

7. The method according to claim 6, further comprising acquiring sound, image and/or video data of the patient or his environment, wherein step b) comprises determining the at least one quantifiable score which can be associated with the quality of life of the patient based on the physiological data and/or at least one of the sound, the image and the video data of the patient or his environment, and the therapy data.

8. The method according to claim 7, wherein determining the at least one quantifiable score which can be associated with the quality of life of the patient based on at least one of the sound, the image and the video data of the patient or his environment, and the therapy data and/or the physiological data comprises processing the sound and/or image data using Natural Language Processing, Sound Processing, Eye Tracking and Processing, Sentiment Analysis using any combination of Convolutional Neural Networks, Transfer Learning, Recurrent Neural Networks, Long-Short-Term Memory Networks, Reinforcement Learning, Auto Encoder and/or Generative Adversarial Networks.

9. The method according to one of the preceding claims, further comprising transmitting the determined at least one quantifiable score to a device accessible by an authorized person and outputting a notification about the determined at least one quantifiable score using the device and/or processing the determined at least one quantifiable score by a computer program.

10. The method according to claim 9, further comprising determining whether one or more of the determined at least one quantifiable score exceeds an upper threshold value and/or is lower than a lower threshold value, and transmitting the one or more of the determined at least one quantifiable score to the device accessible by the authorized person and outputting the notification about the one or more of the determined at least one quantifiable score using the device, and/or processing the one or more of the determined at least one quantifiable score by the computer program, if it is determined that the one or more of the determined at least one quantifiable score exceeds the upper threshold value and/or is lower than the lower threshold value.

11. The method according to claim 10, further comprising determining, based on the acquired physiological data and/or the determination whether one or more of the determined at least one quantifiable score exceeds the upper threshold value and/or is lower than the lower threshold value, whether the patient is in a critical condition, wherein the determined at least one quantifiable score and the one or more of the determined at least one quantifiable score which exceeds the upper threshold value and/or is lower than the lower threshold value, respectively, is transmitted to the device accessible by the authorized person and the notification about the determined at least one quantifiable score and the one or more of the determined at least one quantifiable score which exceeds the upper threshold value and/or is lower than the lower threshold value, respectively, is output using the device and/or the determined at least one quantifiable score and the one or more of the determined at least one quantifiable score which exceeds the upper threshold value and/or is lower than the lower threshold value, respectively, is processed by the computer program, if it is determined that the patient is in the critical condition.

12. The method according to one of claims 9 to 11, wherein the determined at least one quantifiable score and the one or more of the determined at least one quantifiable score which exceeds the upper threshold value and/or is lower than the lower threshold value, respectively, is transmitted to the device accessible by the authorized person and the notification about the determined at least one quantifiable score and the determined one or more of the determined at least one quantifiable score, respectively, is output using the device and/or the determined at least one quantifiable score and the one or more of the determined at least one quantifiable score which exceeds the upper threshold value and/or is lower than the lower threshold value, respectively, is processed by the computer program only if a corresponding consent given by the patient is received.

13. A medical system for automatically monitoring and determining the quality of life of a patient, the medical system comprising:
- at least one medical device (101, 201) having at least one data communication unit and configured to perform a medical therapy on the patient,
- at least one further device (103, 104, 105, 203) having at least one data communication unit, and
- at least one computer program, wherein
the at least one medical device (101, 201) and the at least one further device (103, 104, 105, 203) are configured to exchange data directly or indirectly with each other by means of the respective data communication unit,
the at least one medical device (101, 201) and/or the at least one further device (103, 104, 105, 203) are configured to acquire therapy data concerning the medical therapy that is performed by the at least one medical device (101, 201) on the patient, and
the at least one computer program comprises instructions which, when the program is executed by a computer, causes the computer to determine at least one quantifiable score which can be associated with the quality of life of the patient based on the therapy data.

14. A system for adapting a therapy plan for a patient, comprising a medical system according to claim 13, wherein the at least one computer program comprises instructions which, when the program is executed by the computer, causes the computer to change or optimize a therapy plan of the medical therapy including shortening therapy sessions of the medical therapy and/or changing a kind of the medical therapy depending on the determined at least one quantifiable score and/or the physiological data and/or and at least one of the sound, the image and the video data of the patient or his environment and/or other patient-related data.

15. A system for automatically determining the quality of life of a patient, the system comprising:
- a medical system according to claim 13 or a system according to claim 14,
- at least one computer accessible by authorized personal of a clinic (303) and having a data processing unit and a communication unit,
- at least one computer accessible by an established physician (304) and having a data processing unit and a communication unit,
- at least one computer accessible by a medical device manufacturer (302) and having a data processing unit and a communication unit, and
- a central data processing unit (301) having a data processing unit and a communication unit, wherein
the at least one medical device (101, 102) and/or the at least one further device (103, 104, 105, 203) are configured to transmit the acquired therapy data, the acquired psychological, the acquired sound and/or image data to the central data processing unit (301),
the at least one computer accessible by an established physician (304) is configured to transmit anamnesis data, current test data and/or patient history data of the patient to the central data processing (301),
the at least one computer accessible by authorized personal of a clinic (303) is configured to transmit clinic data regarding the patient to the central processing unit (301),
the at least one computer accessible by a medical device manufacturer (302) is configured to transmit data concerning the at least one medical device (101, 201) to the central processing unit (301),
the central data processing unit is configured to determine the at least one quantifiable score which can be associated with the quality of the patient based on the therapy data and/or the psychological data and/or the sound data and/or the image data transmitted from the at least one medical device (101, 102) and/or the at least one further device (103, 104, 105, 203) and/or the clinic data regarding the patient transmitted from the at least one computer accessible by authorized personal of a clinic (303) and/or the anamnesis data, current test data and/or patient history of the patient transmitted from the at least one computer accessible by an established physician (304) and/or the data concerning the at least one medical device transmitted from the at least one computer accessible by a medical device manufacturer (302).
